(19) Europäisches Patentamt
European Patent Office
Office européen des brevets

(11) Publication number: **0 384 043 A2**

# (12) EUROPEAN PATENT APPLICATION

(21) Application number: 89201637.9

(51) Int. Cl.[5]: C07C 67/287, C07C 37/62

(22) Date of filing: 22.06.89

(30) Priority: 21.02.89 US 313665

(43) Date of publication of application:
29.08.90 Bulletin 90/35

(84) Designated Contracting States:
BE CH DE ES FR GB IT LI NL

(71) Applicant: THE DOW CHEMICAL COMPANY
2030 Dow Center Abbott Road P.O. Box 1967
Midland Michigan 48640-1967(US)

(72) Inventor: Orvik, Jon A.
1164 Lincoln Avenue., //338
Walnut Creek California 94596(US)
Inventor: Pearson, Norman R.
3049 Naranja Drive
Walnut Creek California 94598(US)
Inventor: Haag, Anthony P.
1163 E. Stewart
Midland Michigan 48640(US)
Inventor: Adaway, Timothy J.
3117 Lambros
Midland Michigan 48640(US)
Inventor: Kershner, Larry D.
4405 Oakridge Drive
Midland Michigan 48640(US)
Inventor: Kende, Andrew S.
19 Larchwood Drive
Pittsford New York 14534(US)

(74) Representative: Smulders, Theodorus A.H.J., Ir. et al
Vereenigde Octrooibureaux Nieuwe Parklaan 107
NL-2587 BP 's-Gravenhage(NL)

## (54) Methods for the preparation of brominated intermediates.

(57) A process for preparing substituted 4-bromo phenoxyphenols which are useful in the preparation of herbicidal (phenoxyphenoxy)propionates is disclosed. The process involves the bromination of substituted phenoxy compounds.

EP 0 384 043 A2

## METHODS FOR THE PREPARATION OF BROMINATED INTERMEDIATES

The present invention concerns a process for preparing brominated intermediates useful for making herbicidal (phenoxyphenoxy)propionate derivatives.

Various (phenoxyphenoxy)propionate compounds are known to possess herbicidal and plant-growth regulating properties. For example, see U.S. Patents 3,954,442; 4,550,192; 4,332,961; 4,332,960; 4,370,489; 4,276,080 and British Patent Specification 1,577,181. Also, methods for preparing enantiomers are described in U.S. Patents 4,531,969 and 4,532,328. In U.S. Patent 4,252,980 a method is taught for preparing the methyl ester.

Of particular interest among the (phenoxyphenoxy)propionate herbicides are the 2-(4-(2′-halo-4′-bromophenoxy)phenoxy)propionates which exhibit particularly desirable properties as described in U.S. Patents 4,370,489; 4,531,969; and 4,550,192.

Processes for making selected intermediates used to prepare (phenoxyphenoxy)propionates are taught in U.S. Patents 3,721,703; 4,238,626; 4,252,980; 4,301,295; 4,309,547 and 4,391,995. Although these processes are effective, a number of them utilize diazonium reactions which are potentially explosive when employed on an industrial scale. To avoid the difficulties associated with diazonium reactions, alternative chemistry has recently been developed. This chemistry, based on the Baeyer-Villiger rearrangement, is outlined as follows:

**Step (a): Oxidation**

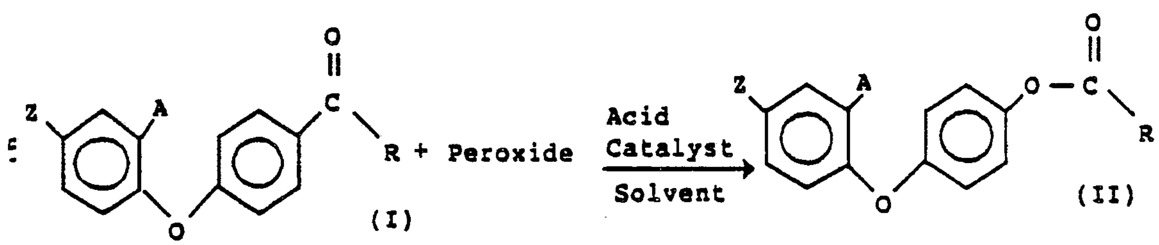

(I) (II)

**Step (b): Hydrolysis/Alcoholysis**

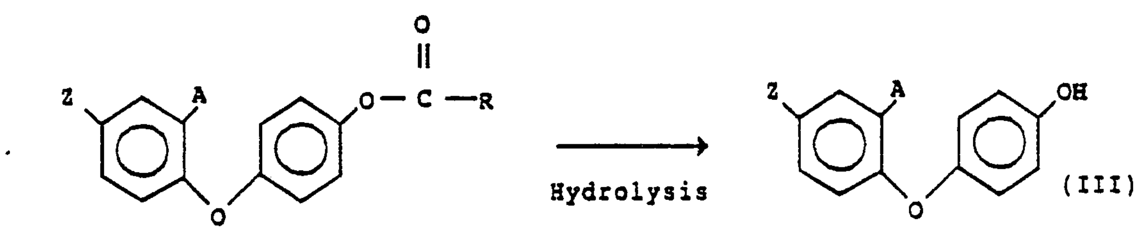

(III)

(II)

wherein

Z is bromine (Br) or hydrogen (H);

A is halogen; and

R is H, C-1 to C-10 alkyl, phenyl or substituted phenyl of the formula

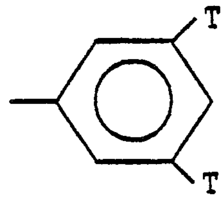

wherein T is halogen or H.

The present invention is directed to preparing brominated intermediates useful for making herbicidal (phenoxyphenoxy)propionate derivatives according to this alternative method.

More specifically, the present invention is directed to selective bromination methods for the preparation of brominated compounds of the formula (V)

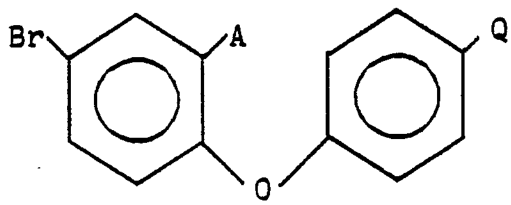

(V)

wherein
Q is
$-O\overset{O}{\overset{\|}{C}}R$ or OH; and
A and R are as previously defined.

As used herein, "halogen" refers to fluorine, chlorine, bromine and iodine.

Such phenoxyphenyl esters and phenoxyphenols are valuable intermediates in the preparation of (phenoxyphenoxy)propionate compounds useful as herbicides and plant growth regulators.

The phenoxyphenone starting materials of Formula (I) can be prepared by a variety of procedures. For example, a substituted phenol can be contacted with a phenylcarbonyl compound in the presence of a base and solvent under conditions effective to give the desired phenoxyphenone of Formula (I). The reaction is exemplified as follows:

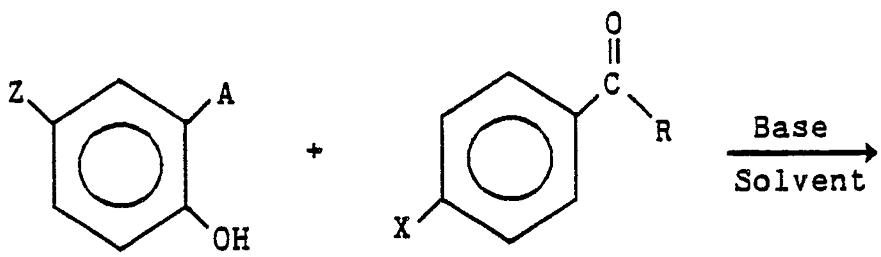


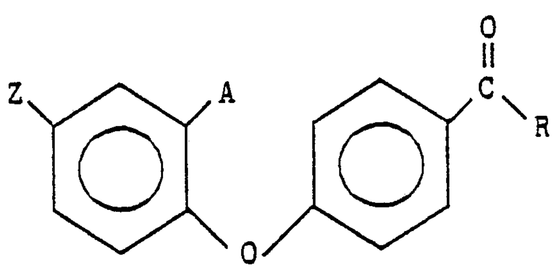

(I)

wherein
Z is Br or H;
A is halogen;

X is halogen; and
R is H, C-1 to C-10 alkyl, phenyl or substituted phenyl of the formula

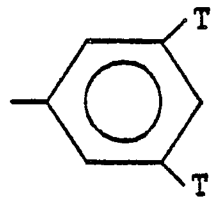

wherein T is independently halogen or H.

With respect to the phenol, Z is preferably hydrogen and A is preferably fluoro or chloro. With respect to the phenylcarbonyl compound, X is preferably fluoro or chloro and R is C-1 to C-10 alkyl or phenyl.

The phenol can be contacted with the phenylcarbonyl compound in molar ratios ranging from stoichiometric (i.e., 1:1) to a slight excess (i.e., 2-3 percent) of either reactant.

Suitable bases for contacting the phenol and the phenylcarbonyl compound include the bases of alkali and alkaline earth metals such as sodium hydroxide (NaOH), potassium hydroxide (KOH), sodium carbonate ($Na_2CO_3$), potassium carbonate ($K_2CO_3$) and the hydroxides and carbonates of magnesium or lithium. Other bases also include sodium or potassium methoxide.

The molar ratio of base to phenol can be in the range from about 1:1 to an excess of about 10:1 (base:phenol), preferably in amounts of slight excess over stoichiometric, e.g., 1.1-1.5:1.

Suitable inert organic solvents which can be employed include polar aprotic solvents such as N-methylpyrrolidone (NMP), dimethylsulfoxide (DMSO), hexamethylphosphoramide (HMPA), sulfolane, acetonitrile or mixtures thereof.

The reactants can be contacted at temperatures ranging from ambient to about 250 degrees Centigrade (°C), preferably from about 50 to 190°C. The reactants are contacted for a time sufficient to ensure substantial completion of the reaction, ranging from about 15 minutes (min) to 24 hours (hr) or more. The contacting is carried out at ambient pressures, although pressures greater than ambient can be employed.

The contacting of the reactants can be carried out in the presence of air, although in order to reduce by-product formation, the contacting is preferably carried out under an inert atmosphere such as that provided by nitrogen, helium or argon, preferably nitrogen.

Antioxidants can be employed in the reaction mixture to reduce by-product formation. Such antioxidants include *t*-butylcatechol, benzothiazine, butylated hydroxytoluene (BHT), butylated hydroxyanisole (BHA) or mixtures thereof. The amount of antioxidant employed in the reaction mixture can range from about 0.1 to about 10 percent (%) by weight of either the phenol (I) or phenyl carbonyl reactant preferably about 2 percent.

The requisite phenols are known compounds and can be prepared, for example, as described in U.S. Patent 4,550,192. Appropriate phenyl carbonyl compounds can be prepared, for example, by the processes taught by N. Buu-Hoi and P. Jacquignon in *Rec. Trav. Chim.* 68, 781-8 (1949) or analogous procedures thereof.

Alternatively, when R is C1-C10 alkyl, phenyl or substituted phenyl of the formula

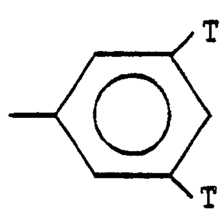

wherein T is independently halogen or H,
the phenoxyphenones of Formula (I) may be prepared by the conventional Friedel-Crafts acylation of the appropriate diphenyl ether with an acyl halide in the presence of a Lewis acid catalyst according to the following reaction:

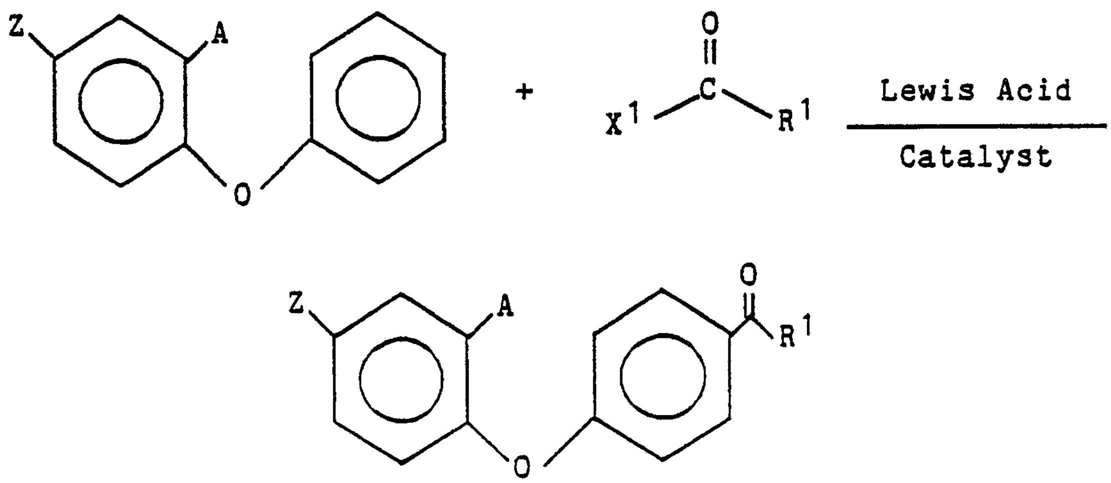


wherein
Z is Br or H;
A is halogen;
$X^1$ is halogen; and
$R^1$ is C1-C10 alkyl, phenyl or substituted phenyl of the formula

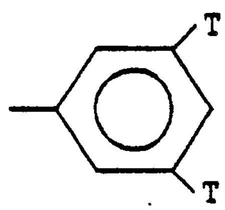

wherein T is independently halogen or H.

With respect to the diphenyl ether, Z is preferably hydrogen and A is preferably fluoro or chloro. With respect to the acyl halide, $X^1$ is preferably chloro or bromo and $R^1$ is preferably substituted phenyl of the formula

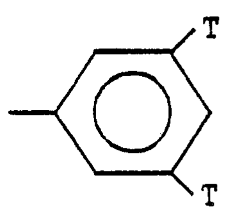

wherein T is independently halogen or H.

The diphenyl ether can be contacted with the acyl halide in molar ratios ranging from stoichiometric (i.e., 1:1) to a slight excess (i.e., 2-3 percent) of either reactant.

Suitable Lewis acid catalysts include the chlorides of aluminum, iron, tin, antimony and boron. A little more than one mole of catalyst per mole of acyl halide is required since the first mole of catalyst coordinates with the oxygen of the acylating reagent.

Suitable inert solvents which can be employed include polar solvents such as nitrobenzene and nonpolar solvents such as methylene chloride, chloroform and carbon tetrachloride.

The reactants can be contacted at temperatures ranging from about 0 to about 200 °C, preferably from about 0 to about 100 °C. The reactants are contacted for a sufficient time to ensure substantial completion of the reaction, ranging from about 15 min to 24 hr or more. The contacting is carried out at ambient pressure, although pressures greater than ambient can be employed.

The requisite diphenyl ether can be prepared, for example, by the Ullmann diaryl ether synthesis. Appropriate acyl halides can be prepared by the reaction between the corresponding acid and an inorganic

acid halide such as, for example, thionyl chloride.

The desired phenoxyphenone (I), prepared from either the phenol and phenylcarbonyl or the diphenyl ether and acyl halide starting materials can be recovered by conventional procedures such as distillation, recrystallization, phase separation or extraction from water into a water-immiscible organic solvent such as methylene chloride, perchloroethylene, carbon tetrachloride ($CCl_4$) or chloroform ($CHCl_3$). The phenoxyphenone can be further purified by repeating the above procedures or can be used in its unpurified form for subsequent reactions.

By following the preparative procedures as set forth in the examples herein and by employing the appropriate starting materials or reactants, the following compounds are prepared, as set forth in Table 1.

Table 1

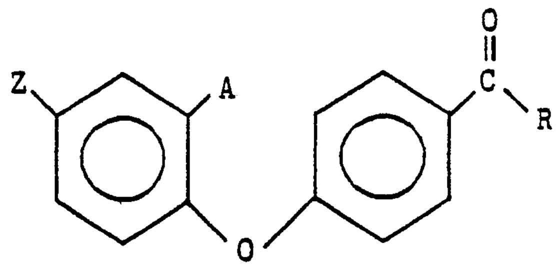

| Z | A | R |
|---|---|---|
| H | H | Φ (phenyl) |
| H | F | Φ |
| H | F | $CH_3$ |
| H | F | H |
| H | F | 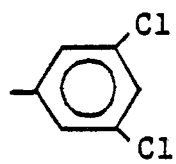 |
| Br | F | Φ |
| Br | F | $CH_3$ |
| Br | F | H |
| H | Cl | Φ |
| H | Cl | $CH_3$ |
| H | Cl | H |
| Cl | Cl | Φ |
| Cl | Cl | $CH_3$ |
| Cl | Cl | H |
| Br | Cl | Φ |
| Br | Cl | $CH_3$ |
| Br | Cl | H |

<u>Step (*a*): Oxidation</u>

In Step (*a*) of the process overall, the phenoxyphenone of Formula (I) is contacted with a peroxide in the presence of an acid catalyst to give the desired phenoxyphenyl ester of Formula (II). The reaction is exemplified as follows:

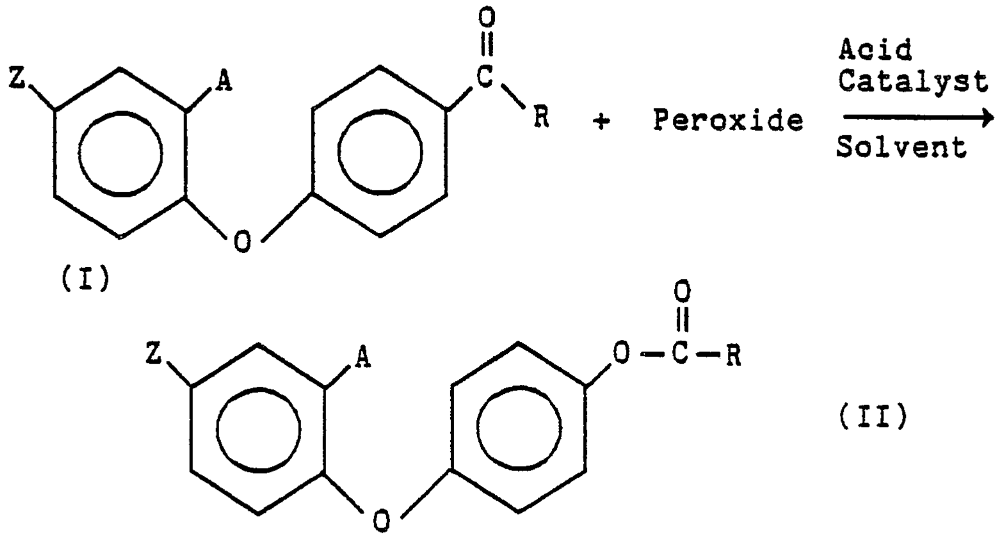


(I)

(II)

wherein Z, A, and R are as defined hereinbefore.

Peroxides which can be employed include hydrogen peroxide (HOOH) alone or peracetic acid ($CH_3COOOH$) alone; the peroxide can also be mixtures of hydrogen peroxide with either acetic acid ($CH_3COOH$) or peracetic acid. Where a mixture of hydrogen peroxide and acetic acid is employed, the acetic acid is employed to regenerate additional peracetic acid for reaction with the phenoxyphenone of Formula (I). The peroxide is employed in amounts ranging from about 1 to about 10, preferably from about 1.1 to about 2.0 moles peroxide to 1 mole phenoxyphenone (I).

Where a mixture of hydrogen peroxide and acetic acid is employed, the ratio of acetic acid to peroxide can vary, ranging from about 100 or more moles acetic acid per mole of peroxide to about 1 mole acetic acid per mole of peroxide; preferably ratios from about 15:1 (moles acetic acid:moles peroxide) to about 2:1 are employed.

In the situation where peroxide is contacted with the phenoxyphenone (I) without acetic acid in amounts sufficient to oxidize the phenoxyphenone (I) to the desired phenoxyphenyl ester (II), such amounts can range from about 10 to about 1.1 moles peroxide per mole phenoxyphenone (I). A slight stoichiometric excess of peroxide in the range of about 1.2-2:1 (moles peroxide:moles phenoxyphenone) is preferred.

The acid catalyst in the process of Step (*a*) can be any acidic catalyst which will catalyze the oxidation of the phenoxyphenone of Formula (I) by the peroxide to yield the phenoxyphenyl ester of Formula (II). Such acid catalysts include mineral acids such as sulfuric acid ($H_2SO_4$), phosphoric acid ($H_3PO_4$) or organic acids such as acetic acid or acidic ion exchange resins such as Dowex® MSC-1-H ion exchange resin (trademark of The Dow Chemical Company).

The acid catalyst is employed in an amount ranging from about 0.1 to 100 moles acid catalyst per mole phenoxyphenone (I), preferably about 1-3:1 (moles acid catalyst:moles phenoxyphenone).

The process of Step (*a*) can be conducted neat although a slight amount to an excess of inert solvent can be employed to dissolve any starting materials in solid or crystalline form. Solvents which can be employed include the C1-C6 alcohols, such as methanol, ethanol, butanol, hexanol, and the like. Other solvents include acetonitrile.

Acetic acid which can serve as both a reactant and as a solvent, can be employed in excess amounts relative to the phenoxyphenone (I).

The process of Step (*a*) is conducted at temperatures, pressures, and times effective to convert the phenoxyphenone of Formula (I) to the phenoxyphenyl ester of Formula (II). Such temperatures can range from about 0 °C to about the temperatures which would cause significant decomposition of the peroxide, preferably from 0 to about 80 °C, more preferably from about 0 to about 60 °C, most preferably from about 20 to 50 °C. The process of Step (*a*) can be conducted at pressures greater than ambient, although ambient pressures are preferred.

The ingredients in Step (*a*) can be contacted in any particular order. Preferably, though, the phenoxyphenone, solvent and acid catalyst of Step (*a*) are first contacted together followed by subsequent additions of small amounts of peroxide, due to the highly exothermic reaction of the peroxide in the reaction medium.

The desired phenoxyphenyl ester of Formula (II) can be recovered by conventional techniques such as

extraction and/or distillation. For example, the reaction medium can be diluted with water and the organic phase containing the desired phenoxyphenyl ester (II) can be separated from the aqueous phase. Further purification can be accomplished by subsequent distillation, crystallization or the like.

Step (*b*): Hydrolysis/Alcoholysis

The phenoxyphenyl ester of Formula (II) can be converted to the desired phenoxyphenol of Formula (III) by three alternative methods.

Alternative 1 - *In-Situ* Acid Hydrolysis

In the *in-situ* acid hydrolysis, the phenoxyphenone (I) is converted directly to the phenoxyphenol (III). The phenoxyphenyl ester (II) is hydrolyzed in the aqueous acidic reaction media without isolation according to the following scheme:

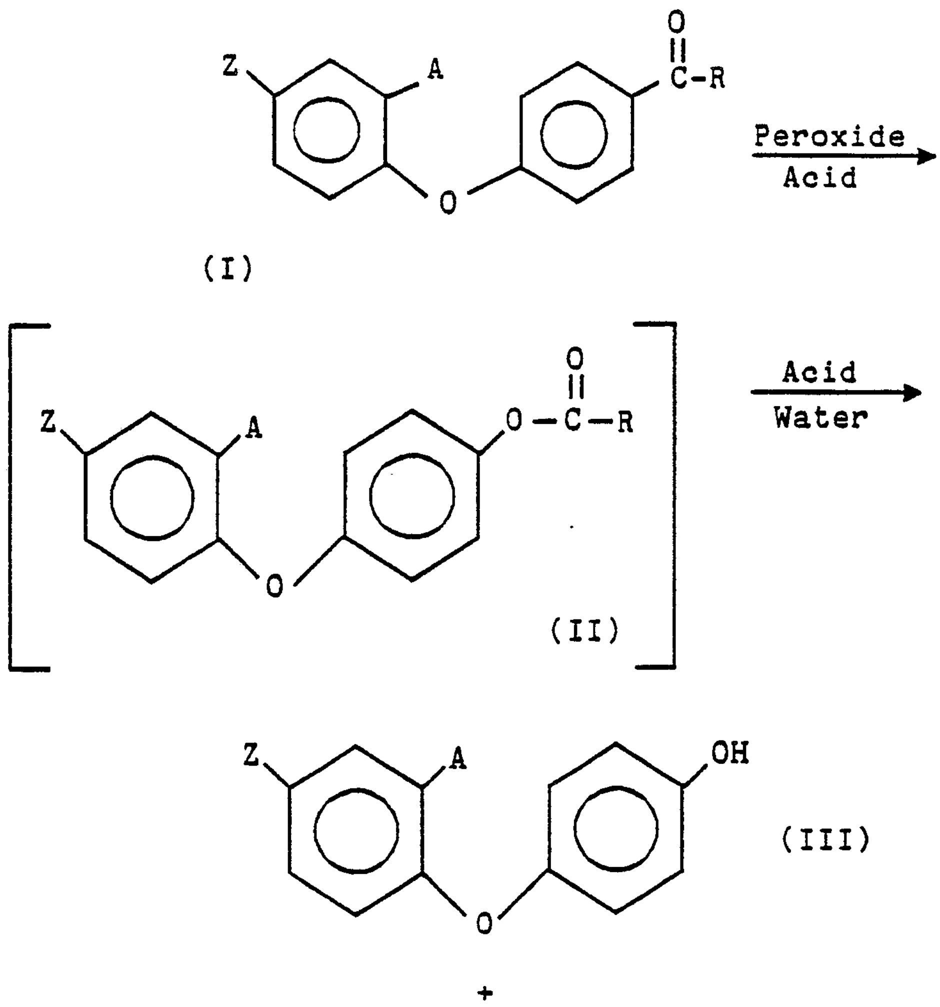


(I)

(II)

(III)

+

RCOOH

wherein Z, A and R are as defined hereinbefore.

The hydrolysis of the phenoxyphenyl ester (II) to the desired phenoxyphenol (III) occurs *in-situ* during Step (*a*) when sufficient acid is present, so that an additional separate contacting step such as in Alternatives 2 and 3 is not essential in order to prepare the desired phenoxyphenol (III). Generally, such amounts can range from stoichiometric to excess acid, preferably a slight excess. Acids which can be employed for hydrolysis include mineral acids such as sulfuric acid ($H_2SO_4$), hydrochloric (HCl), phosphoric

($H_3PO_4$) or organic acids such as acetic acid, toluene sulfonic acid, acidic ion exchange resins, such as Dowex® MSC-1-H.

During Step (*a*) wherein for the phenoxyphenone (I) R is phenyl or

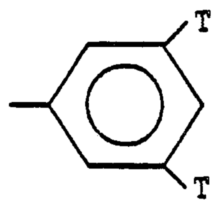

higher temperatures may be required than is needed for the hydrolysis where R is H or alkyl. Excess or residual peroxide need not be removed prior to the hydrolysis, although preferably, any residual or excess peroxide present with the phenoxyphenone (I) and/or phenoxyphenyl ester (II) is neutralized prior to hydrolysis to the phenoxyphenol (III). Any residual peroxide can be neutralized by adding a stoichiometric amount of a reducing agent such as sodium sulfite or sulfur dioxide. Following neutralization of the peroxide, the phenoxyphenyl ester (II) wherein R is phenyl or

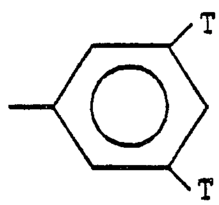

is hydrolyzed *in-situ* by elevating the temperature of the reaction medium to a temperature effective to hydrolyze the phenoxyphenyl ester (III), generally between about 100° to about 200° C.

Recovery of the desired phenoxyphenol (III) prepared from the phenoxyphenone (I) and/or phenoxyphenyl ester (II) wherein R is H or alkyl can be accomplished by extraction procedures. After reaction, water and optionally, a water-immiscible organic solvent is added to the reaction medium, followed by separation of the organic phase containing the desired phenoxyphenol (III) and distillation thereof. Alternatively, the reaction medium can be diluted with water followed by crystallization and filtration to recover the desired phenoxyphenol (III).

Recovery of the desired phenoxyphenol (III) prepared from the phenoxyphenone (I) and/or phenoxyphenyl ester (II) wherein R is phenyl or

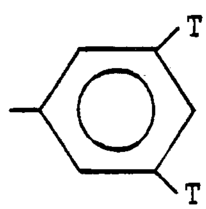

can be accomplished by dilution of the reaction medium with water and separation of the organic phase. The organic phase is washed with a stoichiometric amount of a base in quantities sufficient to convert the residual organic acids such as benzoic acids to their corresponding salts and yet maintain the desired phenoxyphenol (III) in its protonated form. For example, the pH can be adjusted to about 5.5-8.0 and the desired phenoxyphenol (III) can then be recovered by phase separation.

Alternative 2 - Base Hydrolysis

In alternative method 2, the phenoxyphenyl ester (II) is contacted with an aqueous base to give the phenoxyphenate (III′), followed by subsequent contacting with a suitable acid in amounts effective to yield the desired phenoxyphenol (III) as follows:

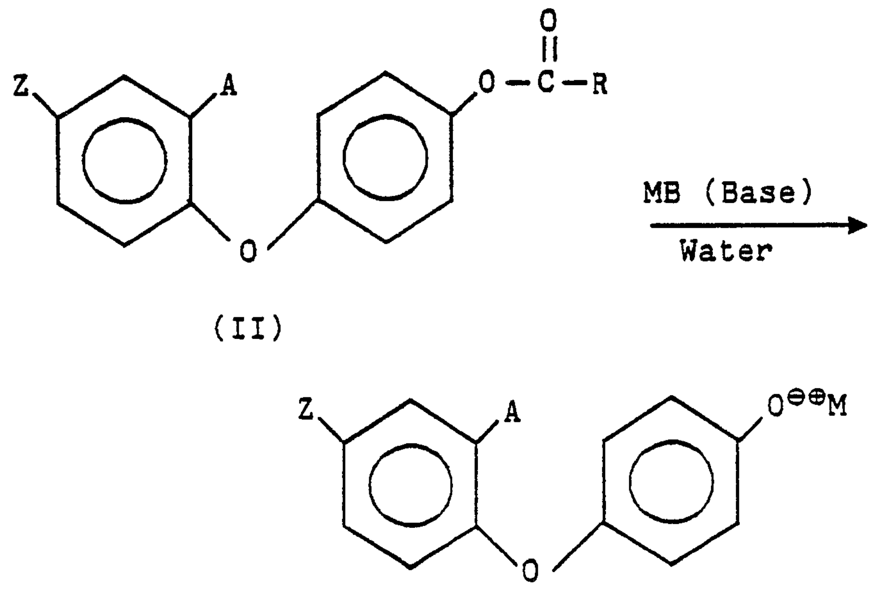


(II)

(III')

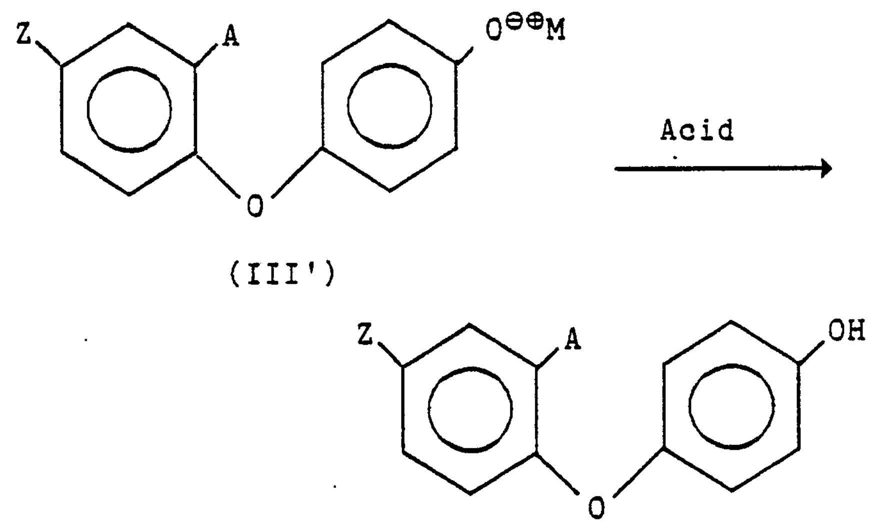


(III')

(III)

wherein Z, A and R are as defined hereinbefore.

The bases (MB) employed for converting the phenoxyphenyl ester (II) to the phenoxyphenate salt (III′) include the carbonate or hydroxide bases of the alkali and alkaline earth metals such as sodium, potassium or lithium; preferably, the hydroxide bases are employed. The base can also be anhydrous sodium or potassium methoxide. A molar excess of base relative to the phenoxyphenyl ester (II) is used to cleave the phenoxyphenyl ester (II) to the phenoxyphenolate (III′). Preferably two or more moles of base are contacted with the phenoxyphenyl ester (II).

Inert solvents can be employed in amounts sufficient to dissolve the phenoxyphenyl ester (II), the base or both. Suitable solvents include non-acidic solvents such as C-1 to C-6 alcohols including methanol, ethanol, propanol, hexanol and the like, aromatic hydrocarbons such as toluene and chlorobenzene; chlorinated hydrocarbons such as methylene chloride, carbon tetrachloride, perchloroethylene and the like; and polar solvents such as dimethylsulfoxide (DMSO), tetrahydrofuran and sulfolane.

The temperatures, pressures and times employed should be sufficient to yield the desired phenoxyphenolate (III′). Such temperatures can range from about 0° to about 100° C, preferably from about

ambient to the melting point of the phenoxyphenyl ester (II). Ambient pressures are preferred although pressures greater than ambient can be employed. The times for contacting of the ingredients can vary from about one hr to 24 hr or more.

The phenoxyphenolate (III′) can be converted to the desired phenoxyphenol (III) by contacting the phenoxyphenolate (III′) with sufficient acid to bring the pH of the reaction medium to a pH less than or equal to about 6. Generally, such amounts can range from stoichiometric to excess acid, preferably a slight excess. Acids which can be employed for converting the phenoxyphenolate (III′) to the desired phenoxyphenol (III) include mineral acids such as $H_2SO_4$, HCl, $H_3PO_4$, or organic acids such as acetic acid, toluenesulfonic acid or acidic ion exchange resins such as those described hereinbefore. The desired phenoxyphenol can be recovered by extraction, filtration and/or phase separation methods described hereinbefore.

Alternative 3 - Alcoholysis (Transesterification)

In another alternative method, the phenoxyphenyl ester (II) is contacted with an alcohol (R′OH) in the presence of a transesterification catalyst to yield the desired phenoxyphenol (III) as follows:

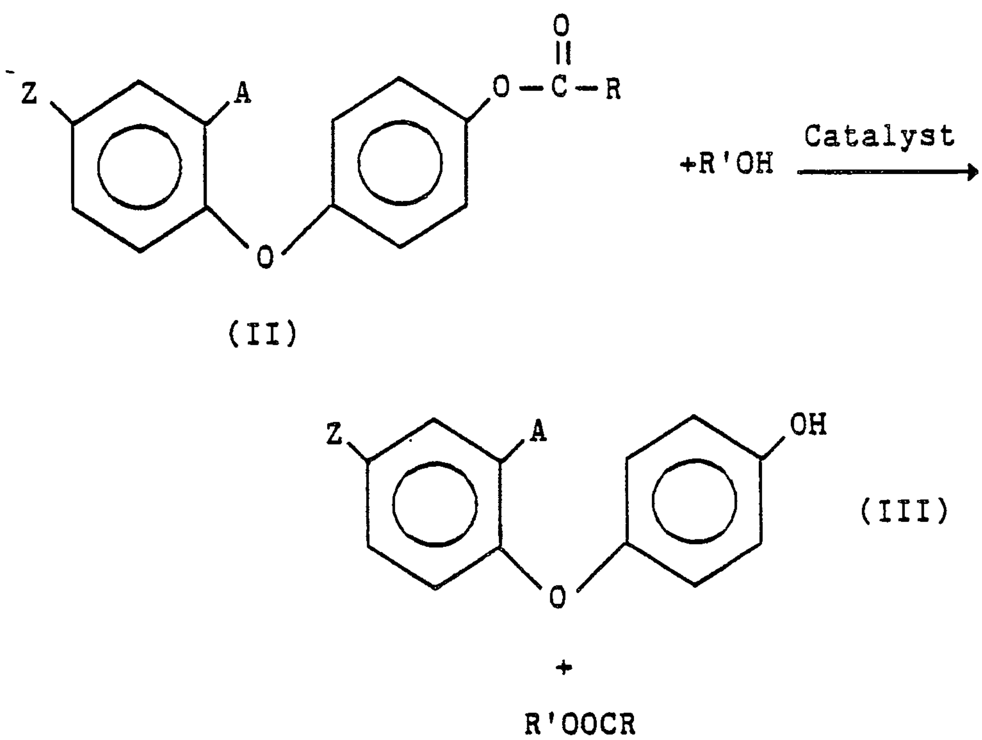


(II)

(III)

wherein

Z, A, and R are as defined hereinbefore; and

R′OH represents C-1 to C-20 primary, secondary and tertiary alcohols, preferably primary alcohols, more preferably C-1 to C-6 primary alcohols, most preferably C-6 alcohol or hexanol.

The alcohol (R′OH) is employed in amounts sufficient to convert the phenoxyphenyl ester (II) to the desired phenoxyphenol (III). Such amounts can range from an excess of alcohol to stoichiometric amounts relative to the phenoxyphenyl ester (II) such as 100-1:1 (moles alcohol:moles phenoxyphenyl ester (II)), preferably about 5-3:1.

Catalysts which can be used to transesterify the phenoxyphenyl ester (II) to the desired phenoxyphenol (III) include mineral acids such as $H_2SO_4$, HCl and $H_3PO_4$; strong organic acids such as toluenesulfonic acid and acidic ion exchange resins described hereinbefore; and preferably tetraalkyltitanate catalysts of the formula:

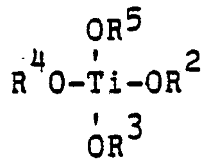

wherein $R^2$, $R^3$, $R^4$ and $R^5$ independently represent C-1 to C-6 alkyl, preferably C-4; most preferably where $R^2$, $R^3$, $R^4$ and $R^5$ each is C-4 alkyl. The catalyst is used in amounts sufficient to convert the phenoxyphenyl ester (II) to the desired phenoxyphenol (III). The amounts of catalyst can range from about 0.1 to about 10.0 percent (weight basis) based on the weight of the phenoxyphenyl ester (II); preferably from about 0.1 to about 2.0 percent; more preferably from about 0.1 to about 0.5 percent by weight.

The temperatures, pressures and times for contacting the ingredients should be sufficient to convert the phenoxyphenyl ester (II) to the desired phenoxyphenol (III). The temperatures can range from about 80 to about 150 °C. Preferably the pressure employed is ambient. However, higher pressures can be employed where alcohols and low boiling point solvents are used, such as for methanol, ethanol, etc. The ingredients are contacted by mixing them for a period ranging from about 0.5 hr to 24 hr or more. The transesterification can occur *in-situ* where the oxidation of step (*a*) is carried out in the presence of an alcoholic solvent ($R'OH$).

The desired phenoxyphenol (III) can be recovered by distillation of excess alcohol and by-products such as alkylbenzoates formed during the reaction. Crystallization and/or filtration procedures can also be employed for recovery.

The phenoxyphenol (III) is converted to the corresponding racemic or optically active (phenoxyphenoxy)propionate by conventional techniques as follows:

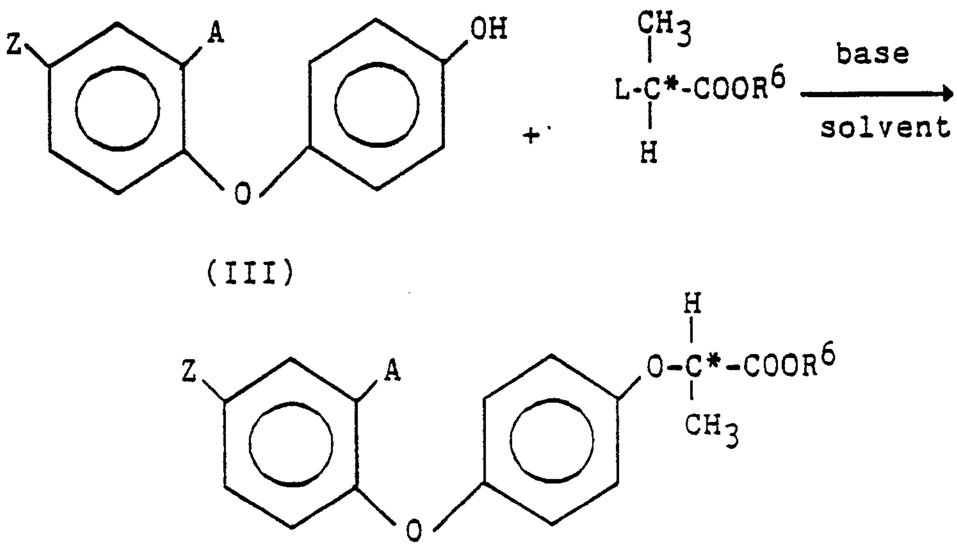


(III)

wherein Z and A are as described hereinbefore;
$R^6$ is hydrogen or C-1 to C-10 alkyl;
L is halogen or an alkyl or aryl sulfonate of the formula

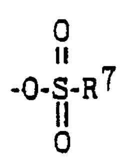

wherein
$R^7$ is C-1 to C-3 alkyl, or phenyl optionally substituted with halogen, C-1 to C-3 alkyl or nitro; and
* represents an asymmetric carbon atom.

In the situation wherein, as to the phenoxyphenyl ester of Formula II and the phenoxyphenol of Formula III, Z is hydrogen, the hydrogen can be converted to bromo before proceeding further in the reaction

sequence. The 4-brominated derivatives can be selectively prepared by contacting a 4-hydrogen derivative of Formula IV with bromine in the presence of a metal halide catalyst and solvent to yield the 4-bromophenoxy derivatives of Formula (V). The reaction is exemplified as follows:

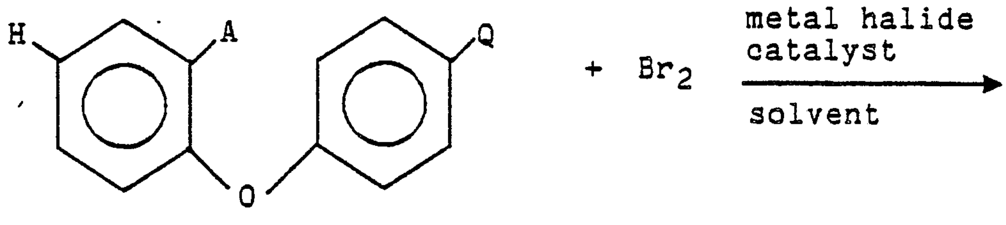


(IV)

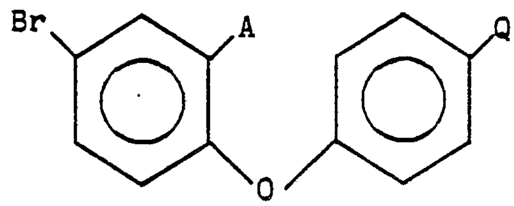

(V)

wherein
Q is
$-O\overset{\displaystyle O}{\overset{\|}{C}}R$ or OH; and
A and R are as previously defined.
The bromination occurs selectively in the ring already containing the halogen (A) and selectively in the 4-position of that ring.

Bromine ($Br_2$) is a non-metallic halogen element which has the property of being a dark, reddish-brown liquid.

The metal halide catalyst is of the formula
$M^nX_n$
wherein
M is aluminum (Al), titanium (Ti), iron (Fe) or boron (B);
X is chloro, bromo or fluoro; and
n is an integer which is the oxidation state of the metal.

Preferably M is aluminum; also preferred is that X is chloro or bromo and that n is 3. For titanium, preferably n is 4. Catalysts which can conveniently be employed include aluminum chloride, aluminum bromide, titanium chloride, titanium bromide, iron chloride, iron bromide and the like; aluminum chloride or bromide are usually preferred.

The metal halide catalyst is used in amounts slightly greater or much greater than the molar equivalents of 4-H-phenoxy derivative (IV) employed. A molar equivalence is defined as one mole of catalyst per mole of 4-H-phenoxy derivative (IV). The amounts of metal halide catalyst can range from 10 to 1.001 moles metal halide catalyst to 1 mole 4-H-phenoxy derivative (IV), more preferably from 1.5-1.1:1 (moles metal halide catalyst:moles 4-H-phenoxy derivative (IV)). The metal halide catalyst should be maintained anhydrous or as water-free as possible, since water can chemically react and deactivate the catalyst.

Contacting of the ingredients is performed in the presence of a solvent resistant or inert to bromination. Such inert solvents include, but are not limited to, chlorinated aliphatic hydrocarbons such as methylene chloride, carbon tetrachloride, chloroform, perchloroethylene, 1,2-dichloroethane, trichloroethylene and the like. The solvent employed can range from an amount sufficient to at least slurry the ingredients up to any amount which would homogenize the ingredients. Typically, the concentration of the 4-H-derivative (IV) is from 5 to 50 percent by weight of a solution containing an inert solvent; preferably from 10 to 20 percent by weight.

The temperatures, pressures and times employed should be sufficient to yield the desired 4-bromophenoxy derivatives of Formula (V). The temperature can range from -30 °C to the boiling point of

bromine, preferably from 0° to 30° C due to the ease of handling the bromine and/or improved selectivity of bromination. The pressure can range from ambient to pressures greater than ambient, preferably ambient pressures. The times for contacting of the ingredients can vary ranging from 15 min to 4 to 5 hr or more. The ingredients can be contacted in any order or by any convenient means. Preferably, the 4-H-phenoxy derivative (IV), solvent and catalyst are first contacted together, followed by subsequent contacting with slow and/or continuous additions of bromine due to the evolution of HBr.

The desired 4-bromophenoxy derivative (V) can be recovered by conventional procedures such as extraction, distillation, recrystallization, filtration and the like.

The Examples which follow illustrate the present invention in a manner by which it can be practiced but, as such, should not be construed as limitations upon the overall scope of the same.

## PREPARATION OF STARTING MATERIALS

Example A - Preparation of 4-(2-fluorophenoxy)benzophenone by coupling 2-fluorophenol and 4-chlorobenzophenone

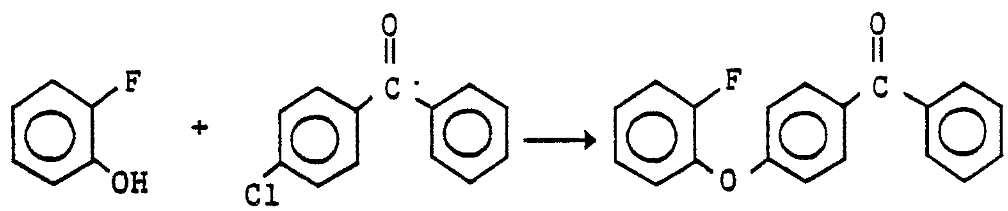

A solution of 224 grams (g) (2.00 moles) of 2-fluorophenol, 263 g of 43 percent by weight aqueous KOH (2.02 moles KOH), and one liter (L) of dimethyl sulfoxide (DMSO) was heated to 125 degrees Centigrade (° C) and water is distilled out with the aid of a Vigreux column under reduced pressure. To the reaction mixture 433 g (2.00 moles) 4-chlorobenzophenone was then added and the resulting mixture was heated for 20 hr at 125° C. The reaction mixture was diluted with 50 milliliters (mL) of concentrated hydrochloric acid (HCl) and 1 liter of water, and then extracted with two 250 mL portions of perchloroethylene. The combined organic phases were washed with 1 liter of water and then concentrated in vacuo to afford 581.2 g of crude product. Purification by distilling out the low boiling impurities was carried out using a column packed with perforated nickel packing. Bottoms product yield was 77 percent for 452.0 g 4-(2-fluorophenoxy)-benzophenone recovered, based on fluorophenol.

An analytical sample has a melting point of 50-52°; $^{13}$C-NMR (75.5 MHz, $CDCl_3$) $\delta$ 161.23 (s), 154.54 (d, $J_{C-F}$-250 Hz), 142.10 (d, J=12 Hz), ,137.84 (s), 132.40 (s), 132.24 (s), 132.09 (s), 132.03 (s), 129.74 (s), 128.20 (s), 126.10 (d, J=8 Hz), 124.96 (d, J=4 Hz), 123.03 (s), 117.31 (d, J=18 Hz), 115.82 (s); ir (neat) 1665 $cm^{-1}$(s), 1605 (s), 1510 (s), 1455 (m), 1420 (m), 1280 (s), 1230 (s).

Example B - Preparation of 4-(2-fluorophenoxy)benzophenone by coupling 2-fluorophenol and 4-fluorobenzophenone

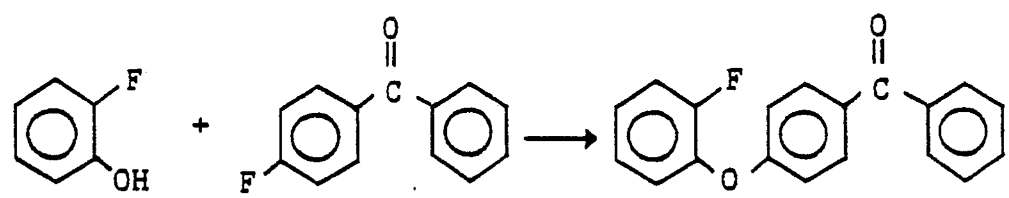

The procedure of Example 1 was employed with the following modifications.

Potassium hydroxide (12.82 g of 86.2 percent KOH; 0.197 moles) was dissolved in 11 mL of $H_2O$ and

added to a solution of 40 g (0.20 moles) 4-fluorobenzophenone in 100 mL of DMSO. The mixture was degassed by three times pulling a vacuum and releasing it with $N_2$. To this solution was added, slowly, 21.5 g (0.19 moles) of *o*-fluorophenol and 2.1 g (0.0095 moles) BHT (2,6-di-*t*-butyl-4-methylphenol). The bulk of the water was removed by vacuum distillation, keeping the pot temperature under 85 °C. The water distillation took 2 hr and 12.2 g of distillate was collected. The reaction mixture, which analyzed at 0.8% $H_2O$, was held at 80 °C for 19 additional hr, at which time conversion was approximately 99 percent. The reaction mixture was diluted with 60 mL $H_2O$, 4 mL conc. HCl, and 20 mL perchloroethylene. The aqueous phase was extracted with an additional 10 mL of perchloroethylene and the combined organic phases were washed with 30 mL $H_2O$. Solvent stripping left 59.5 g of oil, which solidified upon standing. The crude product assayed as 92.0 percent pure 4-(2-fluorophenoxy)benzophenone, for a 98 percent isolated yield.

Example C - Preparation of 4-(2-fluorophenoxy)benzophenone by coupling 2-fluorophenol and 4-fluorobenzophenone

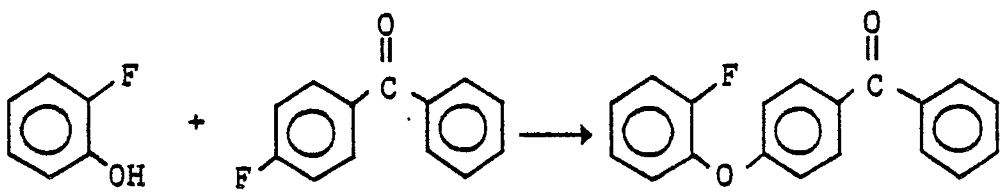

The procedure of Example 1 was employed with the following modifications.

All the components (301 g of 46 percent aqueous KOH, 501 g of fluorobenzophenone, 800 mL of DMSO, and 269 g of fluorophenol), plus 5 mole percent BHT (25 g) were mixed together under a nitrogen atmosphere and distillation of water was carried out at a maximum temperature of 80 °C. Reaction continued at 70 °C for a total of 12 hr. Purification by lights distillation gave an 87 percent yield of 4-(2-fluoro-phenoxy)benzophenone.

Example D - Preparation of 4-(2-fluorophenoxy)phenyl benzoate from 4-(2-fluorophenoxy)benzophenone

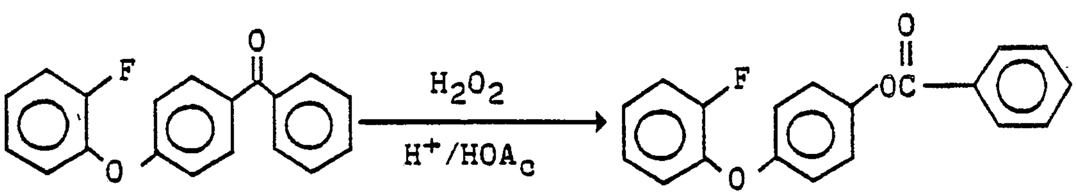


To a 2 liter flask equipped with a thermometer, stirrer and condenser was added 300 g (1.01 moles) of 4-(2-fluorophenoxy)benzophenone, 900 g (15 moles) of acetic acid and 236.2 g (2.4 moles) of sulfuric acid. The reaction mixture was heated to 40 °C and 60 g (1.24 moles) of 70 percent hydrogen peroxide was added over 140 min while maintaining the temperature between 39 to 41 °C; an air gun was used to cool the exothermic reaction. After about half of the hydrogen peroxide had been added, the product began to crystallize. After addition of the hydrogen peroxide was complete, the temperature was held at 40 °C for 7 hr. The reaction mixture was cooled to ambient temperature and was filtered. The solids were washed twice with 350 mL of water. A small portion of the solid 4-(2-fluorophenoxy)phenyl benzoate was dried; m.p. 99.5-101.5 °C.

Example E - Preparation of 4-(2-fluorophenoxy)phenol by base hydrolysis of 4-(2-fluorophenoxy)phenyl benzoate

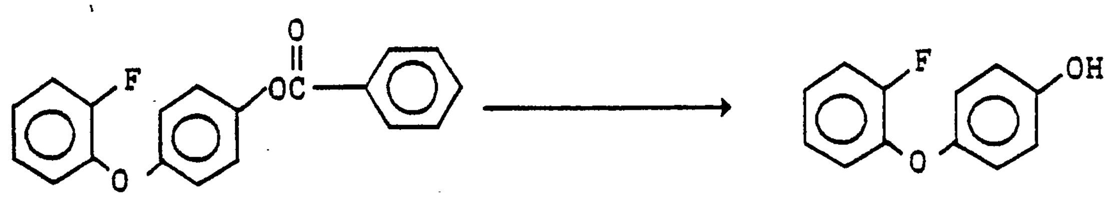

The wet 4-(2-fluorophenoxy)phenyl benzoate of Example 15 was dissolved in approximately 575 mL of perchloroethylene at 60° C and was rapidly mixed with 600 g of 25 percent sodium hydroxide. The reaction mixture was heated to 80° C and monitored by gas chromatography until the hydrolysis was complete. After completion, the reaction mixture was cooled to 60° C and diluted with 250 mL of water. The phases were separated and the aqueous phase was twice washed with 100 mL of perchloroethylene to remove unreacted starting material and other neutral materials from the hydrolysis products which remain in the aqueous phase.

Approximately 660 mL of perchloroethylene was added to the aqueous phase and, while still at 60° C, the pH was slowly adjusted to about 5.9 with about 180 mL of concentrated hydrochloric acid. The organic phase was separated, cooled to about 40° C and mixed with 250 mL of water. The pH of the resulting mixture was adjusted to about 7.8 with about 20 g of 25 percent sodium hydroxide solution. The organic phase containing the 4-(2-fluorophenoxy)phenol was separated from the aqueous phase containing sodium benzoate. The solvent can be evaporated from the organic phase to give 193.5 g of 4-(2-fluorophenoxy)-phenol, m.p. 81.5 to 82° C, or the perchloroethylene extraction solution can be carried forward for bromination without removal of the solvent.

## EXAMPLES OF THE INVENTION

Example 1 - Preparation of 4-(4-bromo-2-fluorophenoxy)phenol from 4-(2-fluorophenoxy)phenol and bromine

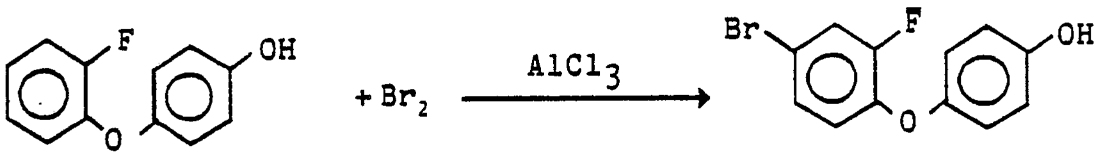


To a 250 mL flask equipped with a nitrogen purge, mechanical stirrer, thermometer, condenser and gas scrubber was added 100 mL of perchloroethylene and 16 g (0.12 moles) of $AlCl_3$. The slurry was heated to 60° C and a solution of 20.4 g (0.10 moles) of 4-(2-fluorophenoxy)phenol in 100 mL of perchloroethylene was added slowly. During the addition HCl gas was given off as the complex with $AlCl_3$ formed. At the end of the addition the temperature was 75° C. The clear, rose-colored solution was cooled to 13° C with an ice-water bath. To the cooled, rapidly stirred solution was added a total of 16 g (0.1 moles) of liquid bromine dropwise over a 1 hr period at 12-16° C. After stirring for an additional 30 min, 80 mL of water and 50 mL of methylene chloride were added and the layers were separated. The organic phase was further extracted with 2-50 mL portions of 10% aqueous HCl and then 2-50 mL portions of water. Stripping the solvent under vacuum gave 27.9 g (98.5%) yield of crude product. This crude product was recrystallized from 20 mL of perchloroethylene to give after precipitation, filtration, and drying, 24.2 g (88% yield) of 4-(4-bromo-2-fluorophenoxy)phenol, mp 81-83.5. The identity to the product was confirmed by $C^{13}$ NMR, IR and mixed mp with an authentic sample.

Example 2 - Preparation of 4-(4-bromo-2-fluorophenoxy)phenyl benzoate by bromination of 4-(2-fluorophenoxy)phenyl benzoate

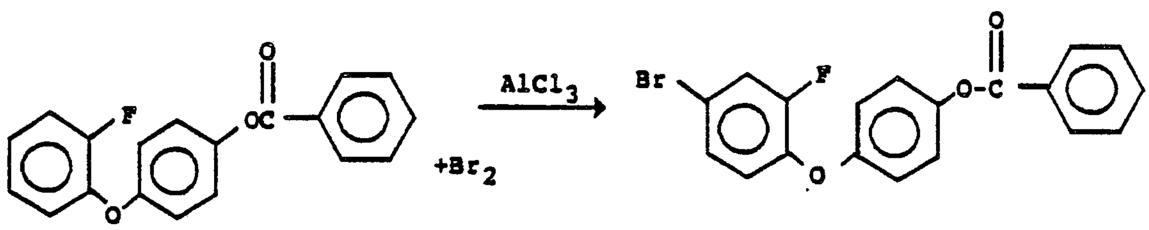


To a 250 mL flask equipped with a nitrogen purge, mechanical stirrer, thermometer, condenser and gas scrubber was added 100 mL of perchloroethylene and 7.2 g (0.054 moles) of $AlCl_3$. The slurry was heated to about 65° C and a solution of 13.87 g (0.045 moles) of 4-(2-fluorophenoxy)phenyl benzoate in 30 mL of perchloroethylene was slowly added. During the addition, HCl gas was given off as the complex with $AlCl_3$ formed. The clear rose-colored solution was cooled to about 20° C, and 7.2 g (0.045 moles) of bromine were added over 1/2 hr to the rapidly stirred solution at 19 to 22° C. After stirring for an additional 30 min, 150 mL of $H_2O$ were added. The organic layer was separated and washed twice with dilute HCl and once with $H_2O$. Evaporation of the solvent gave 17.2 g of product (98% yield). A purified sample has a melting point of 72° to 75° C.

| Elemental analysis: | | |
|---|---|---|
| Calculated for $C_{19}H_{12}BrFO_3$: | C, 58.91; | H; 3.13. |
| Found: | C, 58.85; | H; 3.20. |

## Claims

1. A process for preparing a 4-bromophenoxy derivative of the formula (V):

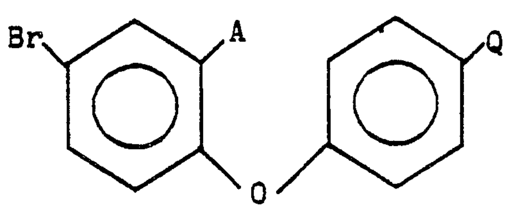

(V)

wherein
Q is
O
||
-O C R or -OH;
A is halogen; and
R is H, C-1 to C-10 alkyl, phenyl or substituted phenyl of the formula

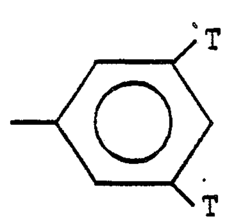

wherein
T is independently halogen or H
which comprises reacting a 4-hydrogen-phenoxy compound of the formula (IV)

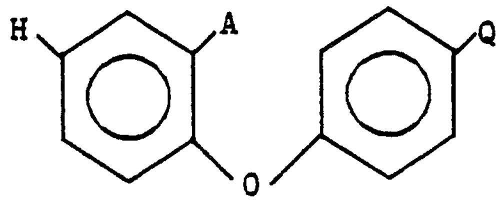

(IV)

wherein A, Q, R and T are defined as above with bromine in the presence of a metal halide catalyst and solvent, wherein the metal halide catalyst is employed in amounts greater than the molar equivalents of 4-hydrogen-phenoxy compound employed, and optionally recovering the desired product.

2. The process of Claim 1 wherein the metal halide catalyst is of the formula

$M^nX_n$

wherein

M is aluminum (Al), titanium (Ti), iron (Fe) or boron (B);

X is chloro, bromo or fluoro; and

n is an integer which is the oxidation state of the metal.

3. The process of Claim 2 wherein M is aluminum, X is chloro or bromo and n is 3.

4. The process of Claim 2 wherein A is fluoro and Q is -OH.

5. The process of Claim 1 wherein the amount of the catalyst is from 1.1 to 1.5 moles of catalyst per mole of 4-hydrogen-phenoxy compound (IV).

6. The process of any one of the preceding Claims wherein the solvent is a chlorinated aliphatic hydrocarbon.

7. The process of Claim 6 wherein the chlorinated aliphatic hydrocarbon is methylene chloride, carbon tetrachloride, chloroform, perchloroethylene, 1,2-dichloroethane or trichloroethylene.

8. The process of Claim 6 or 7 wherein the concentration of 4-hydrogen-phenoxy compound (IV) is from 5 to 50 percent.

9. The process of any one of the preceding Claims wherein the temperature is from -30 to 59 °C.

10. The process of Claim 9 wherein the temperature is from 0 to 30 °C.